# EUROPEAN PATENT SPECIFICATION

(11) **EP 2 995 307 B1**
(45) Date of publication and mention of the grant of the patent: **19.09.2018**
(21) Application number: 14794100.9
(22) Date of filing: 22.04.2014
(51) Int. Cl.: A61K 31/495, A61K 31/205, A61P 39/00, A61P 9/10, A61K 9/00

(54) **ORAL PHARMACEUTICAL COMPOSITION FOR INCREASING HYPOXIA TOLERANCE**
ORALE PHARMAZEUTISCHE ZUSAMMENSETZUNG ZUR ERHÖHUNG DER HYPOXIETOLERANZ
COMPOSITION PHARMACEUTIQUE ORALE DESTINÉE À AMÉLIORER LA TOLÉRANCE À L'ANOXIE

(30) Priority: 06.05.2013 CN 201310161769
(43) Date of publication of application: 16.03.2016
(73) Proprietor: Changzhou Hi-tech District Multiple Dimension Industry Technology Institute CO., LTD., Jiangsu 213022 (CN)
(72) Inventor: XIE, Hebing, Changzhou Jiangsu 213022 (CN); LI, Qingyi, Changzhou Jiangsu 213022 (CN); GU, Shuhua, Changzhou Jiangsu 213022 (CN); LV, Weihong, Changzhou Jiangsu 213022 (CN)
(74) Representative: De Anna, Pier Luigi
(86) International application number: PCT/CN2014/075896
(87) International publication number: WO 2014/180248

(56) References cited:
- WO-A1-97/00678
- CN-A- 102 293 772
- US-A1- 2009 012 096
- PANJWANI U ET AL: "Effect of l-Carnitine Supplementation on Endurance Exercise in Normobaric/Normoxic and Hypobaric/Hypoxic Conditions", WILDERNESS AND ENVIRONMENTAL MEDICINE, ALLEN PRESS, GB, vol. 18, no. 3, 1 September 2007 (2007-09-01), pages 169-176, XP026950597, ISSN: 1080-6032 [retrieved on 2007-09-01]
- DEMAISON ET AL: "Trimetazidine: In vitro influence on heart mitochondrial function", AMERICAN JOURNAL OF CARDIOLOGY, CAHNERS PUBLISHING CO., NEWTON, MA, US, vol. 76, no. 6, 24 August 1995 (1995-08-24), pages 31B-37B, XP005056435, ISSN: 0002-9149, DOI: 10.1016/0002-9149(95)90061-6

## Description

### Technical Field

The present invention relates to pharmaceutical formulations and in particular relates to an oral pharmaceutical composition for use in the treatment of clinical manifestations of hypoxia.

### Background Art

Hypoxia refers to a pathological process in which abnormal changes in metabolism, functions and morphological structures of a tissue occur due to inadequate oxygen supply or dysfunction in oxygen use. Hypoxia consists of 4 types, namely hypotonic hypoxia, hemic hypoxia, circulatory hypoxia and histogenous hypoxia, in which hemic hypoxia and histogenous hypoxia are dysoxidative hypoxia while hypotonic hypoxia and circulatory hypoxia are caused by inadequate oxygen supply.

Hypoxia generates a lot of free radical which damage stability of mitochondrial membrane, hurt body tissues functions and structures, and cause energy metabolism dysfunctions, with clinical manifestation as normal hypoxia manifestation including, among others, dizziness, encephalalgia, tinnitus, dim sight, limb asthenia, lower exercise performance, thought slowness, memory deterioration, nausea, vomit, palpitation, brachypnea, tachypnea and fast but weak heart beat, or as serious diseases including, among other, myocardial infarction, angina pectoris, pneumonedema, encephaledema, shock, respiratory failure, cerebral apoplexy, optic nerve injury and cranial nerves injuries.

Medicines for increasing hypoxia tolerance which is mostly used in clinic are diuretics such as acetazolamide, and adrenocortical hormone agents such as dexamethasone and aminophylline. However, these medicines are not suitable for long-term administration due to their toxic side effect. For example, long term administration of acetazolamide tends to cause adverse effect such as body electrolyte disorder. In addition, traditional Chinese medicine (TCM) preparations comprising Rhodiola rosea are usually used in hypoxia prophylaxis and treatment. These TCM sustained release formulations facilitate enhancement of body adaptability to hypoxia and reduction of stress response, so as to increase hypoxia tolerance. However, these TCMs take effect slowly and provide limited effect. Chinese patent under application number 200310104871.X disclosed that L-carnitine presents effective prophylaxis and treatment of altitude sickness. However, there has been no report on its clinical application so far.

US 2009/0012096 A1 discloses a pharmaceutical composition for reducing the area of myocardial infarction and its use, which composition may comprise L-carnitin and trimetazidine (TMZ).

WO 97/00678 A1 discloses methods related to the treatment of and isolation of compounds for treatment of ischaemic conditions, wherein structural analogues of TMZ may be used for the treatment.

U. Panjwani et al., Wilderness and Environmental Medicine, 2007, volume 18, pages 169 to 176, discloses an effect of L-carnitine supplementation on endurance exercise in normobaric/normoxic and hypobaric/hypoxic conditions.

L. Demaison et al., A Symposium: Management of Myocardial Ischemia*,* discloses that TMZ has an in vitro influence on heart mitochondrial function.

Apparently, a medicine that is suitable for long term administration, combination of prophylaxis and treatment, and effective increase of hypoxia tolerance, without presenting obvious adverse effect, is still in need.

### Summary of Invention

The objective of the present invention is to provide an oral pharmaceutical composition for use in the treatment of clinical manifestations of hypoxia, which composition is clinically convenient, orally administrable and capable of effectively increasing hypoxia tolerance, the pharmaceutical composition comprising active ingredient L-carnitine or a derivative thereof selected from acetyl-L-carnitine and propionyl-L-carnitine, or a pharmaceutically acceptable salt thereof, active ingredient trimetazidine or pharmaceutically acceptable salt thereof, and a pharmaceutically acceptable auxiliary material, and 66-4000:1 is the weight ratio of L-carnitine or derivative thereof or pharmaceutically acceptable salt thereof and trimetazidine or pharmaceutically acceptable salt thereof. The clinical manifestation of hypoxia include dizziness, encephalalgia, tinnitus, dim sight, limb asthenia, lower exercise performance, thought slowness, memory deterioration, nausea, vomit, palpitation, brachypnea, tachypnea and fast but weak heart beat, pneumonedema, encephaledema, cerebral apoplexy, shock, respiratory failure, optic nerve injury and cranial nerves injuries.

During extensive animal experiments, researchers of the present invention unexpectedly found that trimetazidine or pharmaceutically acceptable salt thereof and L-carnitine or derivative thereof or pharmaceutically acceptable salt thereof can be combined in a predetermined proportion in administration or into a composition, which can increase blood oxygen saturation of hypoxic rats and extend the survival period of mice in hypoxic condition.

Researchers of the present invention prepared oral pharmaceutical formulations, such as oral tablets, granules and oral liquid, with trimetazidine or pharmaceutically acceptable salt thereof, L-carnitine or derivative thereof or pharmaceutically acceptable salt thereof and a specific pharmaceutically acceptable auxiliary material in a predetermined weight proportion.

Hypoxia refers to a pathological condition in which abnormal changes in metabolism, functions and morphological structures of a tissue occur due to inadequate oxygen supply or dysfunction in oxygen use. In the present invention, hypoxia particularly refers to a pathological condition in which abnormal changes in metabolism, functions and morphological structures of a tissue occur due to inadequate oxygen supply.
In the present invention, clinical manifestation of hypoxia includes normal hypoxia manifestation including, among others, dizziness, encephalalgia, tinnitus, dim sight, limb asthenia, lower exercise performance, thought slowness, memory deterioration, nausea, vomit, palpitation, brachypnea, tachypnea and fast but weak heart beat, pneumonedema, encephaledema, cerebral apoplexy, shock, respiratory failure, optic nerve injury and cranial nerves injuries.
In the present invention, increasing hypoxia tolerance refers to prophylaxis and treatment of symptoms and diseases with clinical manifestation of hypoxia, and in particular refers to prophylaxis and treatment of normal hypoxia manifestation including, among others, dizziness, encephalalgia, tinnitus, dim sight, limb asthenia, lower exercise performance, thought slowness, memory deterioration, nausea, vomit, palpitation, brachypnea, tachypnea and fast but weak heart beat.

The present invention provides an oral pharmaceutical composition for use in the treatment of clinical manifestations of hypoxia, the pharmaceutical composition comprising active ingredient L-carnitine or derivative thereof or pharmaceutically acceptable salt thereof, active ingredient trimetazidine or pharmaceutically acceptable salt thereof, and a pharmaceutically acceptable auxiliary material, and 66-4000:1, preferably 100:1 is the weight ratio of L-carnitine or derivative thereof or pharmaceutically acceptable salt thereof and trimetazidine or pharmaceutically acceptable salt thereof.

In the oral pharmaceutical composition for use according to the present invention, the L-carnitine or derivative thereof or pharmaceutically acceptable salt thereof is selected from L-carnitine, acetyl-L-carnitine, propionyl-L-carnitine and pharmaceutically acceptable salts thereof, and is preferably L-Carnitine; the pharmaceutically acceptable salts of trimetazidine, L-carnitine or derivatives thereof comprise their salts formed with hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid and p-toluene sulfonic acid.

A particularly preferred example of the oral pharmaceutical composition for use according to the present invention is a tablet which comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

A particularly preferred example of the oral pharmaceutical composition for use according to the present invention is a granule which comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

A particularly preferred example of the oral pharmaceutical composition for use according to the present invention is an oral liquid which comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

The oral pharmaceutical composition for use according to the present invention is a formulation for oral administration, including granules, tablets, capsules, oral liquid, preferably tablets, granules and oral liquid. The oral pharmaceutical composition can also use combined package.

### Embodiment

The following examples are provided for further explaining the present invention only and do not intend to limit the scope of the invention.

### Example 1: Observation of influence of oral administration of different dosage combination of L-carnitine+trimetazidine to hypoxic mice in normal pressure

trimetazidine hydrochloride: 0.15, 0.75, 1.5, 3, 6 and 9 mg/kg, equivalent to human daily dosage of 1, 5, 10, 20, 40 and 60mg;
L-carnitine: 600mg/kg, equivalent to human daily dosage of 4g;
70 male mice are selected, each of a weight of 20±2g. The mice are divided into 10 groups randomly based on weight, 10 for each group, and are given oral administration at a dosage of 20ml/kg, while the control group is given isovolumetric normal saline, both once a day for consecutive 7 days. In one hour after the final administration, each group of mice are placed in wide mouth bottles of a volume of 160ml, into which 5g of soda lime has been pre-added. One bottle contains one mouse and its cap is sealed with Vaseline. Taking death of mice as index, putting down the survival time of mice and taking a 20% or more extension of survival time as significant effect. Please refer to table 1 for the results.

**Table 1 Comparison of Survival Time under Normal Pressure in Hypoxic Condition**

| (n=10, *x̅* ± *S*) | | |
|---|---|---|
| Group/Dosage (mg/kg) | Survival Time (min) | Extended Survival Time (%) |
| Control Group | 21.5±5.6 | - |
| L-carnitine 600 + trimetazidine hydrochloride 0.15 | 29.0±2.1 | 34.9 |
| L-carnitine 600 + trimetazidine hydrochloride 0.75 | 29.5±2.9 | 37.2 |
| L-carnitine 600 + trimetazidine hydrochloride 1.5 | 30.4±3.3** | 41.4 |
| L-carnitine 600 + trimetazidine hydrochloride 3 | 31.8±1.4* | 47.9 |
| L-carnitine 600 + trimetazidine hydrochloride 6 | 35.4±4.5** | 64.7 |
| L-carnitine 600 + trimetazidine hydrochloride 9 | 35.0±5.7** | 62.8 |

| | | |
|---|---|---|
| Remarks: as compared to the control group, *P<0.05, **P<0.01. | | |

The results show that: the composition of L-carnitine and trimetazidine hydrochloride (66-40000:1) can extend the survival time of mice in hypoxia and lower weight ratio presents more significant effect. The most significant effect is achieved when the weight ratio of L-carnitine and trimetazidine hydrochloride is 100:1.

### Example 2: Comparison of L-carnitine 600mg/kg + trimetazidine hydrochloride 6mg/kg and separate intragastric administration on mice in hypoxia under atmospheric pressure

40 male mice are selected, each of a weight of 20±2g. The mice are divided into 4 groups randomly based on weight, 10 for each group, and are given intragastric administration at a dosage of 20ml/kg, while the control group is given isovolumetric normal saline, both once a day for consecutive 7 days. In one hour after the final administration, each group of mice are placed in wide mouth bottles of a volume of 160ml, into which 5g of soda lime has been pre-added. One bottle contains one mouse and its cap is sealed with Vaseline. Taking death of mice as index and putting down the survival time of mice. Please refer to table 2 for the results.

**Table 2 Comparison of Survival Time under Normal Pressure in Hypoxic Condition**

| (n=10, *x̅*±*S*) | | |
|---|---|---|
| Group/Dosage (mg/kg) | Survival Time (min) | Extended Survival Time (%) |
| Control Group | 21.5±5.6 | - |
| trimetazidine hydrochloride 6 | 26.0±5.2* | 20.9 |
| L-carnitine 600 | 28.6±4.8* | 33.0 |
| trimetazidine hydrochloride 6+ L-carnitine 600 | 36.0±7.1** | 67.5 |

| | | |
|---|---|---|
| Remarks: as compared to the control group, *P<0.05, **P<0.01. | | |

The results show that: as compared to separate use of L-carnitine or trimetazidine hydrochloride, the composition significantly extends the survival time of mice (P<0.01) Combination of the two medicines provides synergistic effect. Therefore, it shows that compound preparation is better than single preparation.

### Example 3: Observation of influence of administration of different dosage combination of L-carnitine+trimetazidine to hypoxic rats in normal pressure

trimetazidine hydrochloride: 2, 4 and 6 mg/kg, equivalent to human daily dosage of about 20, 40 and 60mg;
L-carnitine: 200, 400 and 600mg/kg, equivalent to human daily dosage of about 2, 4 and 6g;
70 Wister rats are selected, each of a weight of 150g^{∼}190g. The rats are divided into 7 groups randomly: normoxic control group: raised and collected in plain area; acute hypoxia group: animals are placed in a low pressure oxygen cabin having a cabin oxygen partial pressure of 11.01Kpa (equivalent to about oxygen partial pressure at 5000m above sea level). In decompression hypoxia for 3 days, the animals are further placed in a low pressure oxygen cabin having a cabin oxygen partial pressure of 13.25Kpa (equivalent to about oxygen partial pressure at 4000m above sea level) for sampling [Yue ZHENG, Yang JI, Animal Models Commonly Used in Researches for Increasing Hypoxia Tolerance and Medicines for Increasing Hypoxia Tolerance, Pharm J Chin PLA, 2010, 26(2):170-173]; Administration Group: intragastric administration at a dosage of 20ml/kg for seven days since four days before entering the low pressure oxygen cabin. Collecting data and samples in a low pressure oxygen cabin having a cabin oxygen partial pressure at 13.25Kpa (equivalent to about oxygen partial pressure at 4000m above sea level). All animals are freely eating and drinking.

Hemodynamic measurement: at corresponding time point, cardiac catheters are inserted to pulmonary artery via right external jugular vein and to aorta and left ventricle via left common carotid artery of each group of animals; a four-channel physiology recorder is used to record heart rate (HR), pulmonary artery pressure (PAP), systolic aortic pressure (SAP), diastolic aortic pressure (DAP), left ventricle systolic pressure (LVSP), left ventricle diastolic pressure (LVEDP), and the maximum increase rate of left ventricle pressure (+dp/dtₘₐₓ). Please refer to table 3 for the results.

**Table 3 Influence of different dosage combination of L-carnitine and trimetazidine to haematological index of rats in simulated plateau hypoxia condition (n=10, x̅±S)**

| oup/Dosage (mg/kg) | PAP (Kpa) | SAP (Kpa) | DAP (Kpa) | LVSP (Kpa) | +dp/dtₘₐₓ (KPa) | HR (heat/min) |
|---|---|---|---|---|---|---|
| Normoxic Control Group | 3.5 ± 0.6 | 15.8±1.6 | 10.5 + 2.8 | 16.9 + 1.6 | 664±83 | 360±40 |
| Acute Hypoxia Group | 5.3 ± 0.7 | 22.9±3.7 | 15.6 ± 3.2 | 25.5 ± 3.0 | 695±72 | 377±50 |
| L-carnitine 200 + trimetazidine hydrochloride 4 | 4.7 ± 0.8* | 19.7 ± 2.3* | 13.6 ± 3.5* | 21.9 ± 2.8* | 623 ± 77* | 375±52 |
| L-carnitine 400 + trimetazidine hydrochloride 4 | 4.2 ± 0.7** | 18.2 ± 2.5** | 12.5 + 2.6** | 20.1 ± 2.5** | 561 ± 43** | 370+45 |
| L-carnitine 600 + trimetazidine hydrochloride 4 | 3.9 ± 0.5** | 17.5 + 1.9** | 11.2 ± 2.4** | 18.5 ± 1.8** | 517 + 60** | 368±48 |
| L-carnitine 600 + trimetazidine hydrochloride 6 | 3.6 ± 0.5** | 16.1 ± 3.4** | 10.8 ± 2.1** | 17.2 ± 1.9** | 494 ± 54** | 365±50 |
| L-carnitine 600 + trimetazidine hydrochloride 2 | 5.1 ± 0.6* | 20.9 ± 3.5* | 14.3 ± 3.1* | 23.2 ± 3.2* | 657 ± 75* | 375±58 |

| | | | | | | |
|---|---|---|---|---|---|---|
| Remarks: as compared to the acute hypoxia group, *P<0.05, **P<0.01. | | | | | | |

Blood gas analysis: collecting 1 ml of blood from aorta; heparin anticoagulation; measuring blood gas index including, among others, blood oxygen partial pressure PaO₂ and oxygen saturation SaO₂. Please refer to table 4 for the results.

**Table 4 Influence of different dosage combination of L-carnitine and trimetazidine to blood gas analysis of rats in simulated plateau hypoxia condition (n=10, x̅±S)**

| Group/Dosage (mg/kg) | PaO₂ (Kpa) | SaO₂ (%) |
|---|---|---|
| Normoxic Control Group | 12.2±2.4 | 91.4±6.3 |
| Acute Hypoxia Group | 5.5±1.5 | 63.7±13.8 |
| L-carnitine 200 + trimetazidine hydrochloride 4 | 6.9±1.9** | 72.5±13.2** |
| L-carnitine 400 + trimetazidine hydrochloride 4 | 8.2±2.0** | 83.6±10.1** |
| L-carnitine 600 + trimetazidine hydrochloride 4 | 8.1±1.5** | 78.9±16.5** |
| L-carnitine 600 + trimetazidine hydrochloride 6 | 9.8±2.5** | 90.7±14.8** |
| L-carnitine 600 + trimetazidine hydrochloride 2 | 6.3±1.5** | 70.4±11.6** |

| | | |
|---|---|---|
| Remarks: as compared to the acute hypoxia group, **P<0.01. | | |

### The results show that:

According to Table 3, all the Administration Groups can significantly increase hemodynamic indexes, which shows that it has the effect of increasing hypoxia tolerance, and the effect of the combination of L-carnitine 600mg/kg and trimetazidine hydrochloride 6mg/kg is the closest to that of the normoxic control group.

According to Table 4, each administration group can significantly increase arterial blood oxygen partial pressure and oxygen saturation of hypoxic rats (P<0.01), which shows that the composition according to the present invention can increase bonding strength of hemoglobin and oxygen, oxygen carrying capacity and hypoxia tolerance. The effect of the combination of L-carnitine 600mg/kg and trimetazidine hydrochloride 6mg/kg is the closest to that of the normoxic control group.

### Example 4: Study of selection of auxiliary material of normal tablets

Based on physiochemical properties of L-carnitine and characteristics of the dosage form, namely L-carnitine is a flaky crystal and extremely easy to absorb moisture, a sustained release auxiliary material that has moisture absorption resistance shall be adopted. The inventors selected auxiliary materials including, among other, microcrystalline cellulose, calcium carbonate, cross-linked polyvinylpyrrolidone and talcum powder via a great amount of Pharmaceutics Experiments, among which microcrystalline cellulose and calcium carbonate are excipients, cross-linked polyvinylpyrrolidone is a disintegrant, and talcum powder can be used as framework material to increase formability of granules and tablets and as lubricant to avoid sticking and picking during the process of tableting.

Microcrystalline cellulose and calcium carbonate are preferred excipients and their weight ratio directly determines compressibility of tablets. The inventors, by observing actual formulation development process, based on fixed ratio of active ingredients and other auxiliary materials, carefully studied differences in formability of granules/formability of tablets when key auxiliary materials microcrystalline cellulose and calcium carbonate are in different ratios and finally determined a range of ratios of microcrystalline cellulose and calcium carbonate. Please refer to Table 5 for the results.

**Table 5 Table of formability study of different ratios of microcrystalline cellulose and calcium carbonate**

| microcrystalline cellulose : calcium carbonate | Granule formability | Tablet formability |
|---|---|---|
| 5:1 | loose particles which can be sifted through mesh easily after drying; much fine powder of 20%^{∼}30% of the total amount | Tablet formable but sticking and picking occur very easily; strict requirement on environmental humidity under 50% |
| 2:1 | loose particles which can be sifted through mesh easily after drying; much fine powder of 15%^{∼}20% of the total amount | Tablet formable but sticking and picking occur very easily; strict requirement on environmental humidity under 50% |
| 1:1 | loose particles which can be sifted through mesh easily after drying; suitable size with less fine powder of 2%^{∼}6% of the total amount | Tablet formable without sticking or picking; no requirement on environmental humidity under 50% |
| 1:2 | Particle agglomerate which can be sifted through mesh after drying; suitable size with less fine powder of 5%^{∼}10% of the total amount | Tablet formable without sticking or picking; strict requirement on environmental humidity under 50% |
| 1:5 | Particle agglomerate which can hardly be sifted through mesh after drying; much fine powder of over 20% of the total amount | Tablet formable with sticking or picking; strict requirement on environmental humidity under 50% |

The results show that tablets are formable when the weight ratio of microcrystalline cellulose and calcium carbonate is between 5:1 and 1:5, but the weight ratio of microcrystalline cellulose and calcium carbonate is preferably 1:1 for the purpose of easier control.

### Example 5: Study of selection of auxiliary material of granules

Based on physiochemical properties of L-carnitine and characteristics of the dosage form, namely L-carnitine is a flaky crystal and extremely easy to absorb moisture, a sustained release auxiliary material that has moisture absorption resistance shall be adopted. The inventors selected auxiliary materials including, among other, lactose, mannitol, ethanol and citric acid via a great amount of Pharmaceutics Experiments, among which lactose and mannitol are excipients, enthanol is a binding agent, and citric acid is a corrective agent.

Lactose and mannitol are preferred excipients. The inventors, by observing actual formulation development process, based on fixed ratio of active ingredients and other auxiliary materials, carefully studied differences in formability of granules/formability of tablets when key auxiliary materials lactose and mannitol are in different ratios and finally determined a range of ratios of lactose and mannitol. Please refer to Table 6 for the results.

**Table 6 Table of formability study of different ratios of lactose and mannitol**

| lactose : mannitol | Granule formability |
|---|---|
| 5:1 | particles can easily get agglomerated and cannot be easily dispersed after drying; difficult to granulate |
| 2:1 | loose particles can easily be dispersed after drying; easy to granulate; fine powders is 3%^{∼}5% of the total amount |
| 1:1 | loose particles can easily be dispersed after drying; easy to granulate; fine powders is 5%^{∼}10% of the total amount |
| 1:2 | loose particles can easily be dispersed after drying; easy to granulate; fine powders is 10%^{∼}20% of the total amount |
| 1:5 | particles can easily get agglomerated and cannot be easily dispersed after drying; difficult to granulate |

The results show that granules are formable when the weight ratio of lactose and mannitol is between 5:1 and 1:5, but the weight ratio of lactose and mannitol is preferably 2:1 for the purpose of easier preparation.

### Example 6: Study of selection of auxiliary material of oral liquid

Based on the characteristics of the dosage form oral liquid and the physiochemical property that L-carnitine has fishlike smell, selected auxiliary materials are mainly corrective agents and sweeteners, including, among others, sodium cyclamate and citric acid. The ratio of sodium cyclamate and citric acid is determined via taste identification by lab personnel. Please refer to table 7 for the results.

**Table 7 Table of taste study of different ratios of sodium cyclamate and citric acid**

| sodium cyclamate : citric acid | fishlike | sweet | sour |
|---|---|---|---|
| 5:1 | No | Weak | Strong |
| 2:1 | No | Moderate | Strong |
| 1:1 | No | Moderate | Moderate |
| 1:2 | No | Strong | Moderate |
| 1:5 | Yes | Strong | Weak |

The results show that the weight ratio of sodium cyclamate and citric acid between 5:1 and 1:5 provides no irritant taste, but the weight ratio of sodium cyclamate and citric acid is preferably 1:1 for the purpose of best taste.

### Example 7: Compound preparation L-carnitine Tablet

### Formulation (percentage by weight):

L-carnitine: 16%
trimetazidine hydrochloride: 10%
microcrystalline cellulose: 50%
calcium carbonate: 10%
cross-linked polyvinylpyrrolidone: 4%
polyvinylpyrrolidone: 5%
talcum powder: 4%
magnesium stearate: 1%

### Process:

1) Let L-carnitine, trimetazidine hydrochloride, microcrystalline cellulose, calcium carbonate, cross-linked polyvinylpyrrolidone, polyvinylpyrrolidone and talcum powder be sifted through 100 mesh sieve, respectively, for further use;
2) Weighing according to the formulation L-carnitine, trimetazidine hydrochloride, microcrystalline cellulose, calcium carbonate and polyvinylpyrrolidone, which are then evenly mixed;
3) Adding an appropriate amount of 80% ethanol solution, preparing soft material and have it sifted through 20 mesh sieve to prepare granules;
4) Placing wet granules in a forced air drier at 50°C and have them dried for four hours;
5) After drying is complete, the granules are taken out and sifted through 20 mesh sieve for granulation. Then adding prescribed amount of magnesium stearate, talcum powder and cross-linked polyvinylpyrrolidone, which are then mixed evenly;
6) Selecting appropriate punch according to requirement on tablet weight and tableting.

### Example 8: Compound preparation L-carnitine Tablet

### Formulation (percentage by weight):

L-carnitine: 80%
trimetazidine hydrochloride: 0.1%
microcrystalline cellulose: 6%
calcium carbonate: 3%
cross-linked polyvinylpyrrolidone: 2%
polyvinylpyrrolidone: 4%
talcum powder: 4%
magnesium stearate: 0.9%

### Process:

The same as that in example 7

### Example 9: Compound preparation L-carnitine Tablet

### Formulation (percentage by weight):

L-carnitine: 65%
trimetazidine hydrochloride: 10%
microcrystalline cellulose: 5%
calcium carbonate: 10%
cross-linked polyvinylpyrrolidone: 2%
sodium carboxymethyl cellulose: 2%
talcum powder: 5%
magnesium stearate: 1%

### Process:

1) Let L-carnitine, trimetazidine hydrochloride, microcrystalline cellulose, calcium carbonate, cross-linked polyvinylpyrrolidone, sodium carboxymethyl cellulose and talcum powder be sifted through 100 mesh sieve, respectively, for further use;
2) Weighing according to the formulation L-carnitine, trimetazidine hydrochloride, microcrystalline cellulose, calcium carbonate and sodium carboxymethyl cellulose, which are then evenly mixed;
3) Adding an appropriate amount of 70% ethanol solution, preparing soft material and have it sifted through 20 mesh sieve to prepare granules;
4) Placing wet granules in a forced air drier at 50°C and have them dried for four hours;
5) After drying is complete, the granules are taken out and sifted through 20 mesh sieve for granulation. Then adding prescribed amount of magnesium stearate, talcum powder and cross-linked polyvinylpyrrolidone, which are then mixed evenly;
6) Selecting appropriate punch according to requirement on tablet weight and tableting.

### Example 10: Compound preparation L-carnitine Tablet

### Formulation (percentage by weight):

L-carnitine: 65%
trimetazidine hydrochloride: 0.2%
microcrystalline cellulose: 4%
calcium carbonate: 20%
cross-linked sodium carboxymethyl cellulose: 2%
sodium carboxymethyl cellulose: 2%
talcum powder: 5%
magnesium stearate: 0.8%

### Process:

The same as that in example 9

### Example 11: Compound preparation L-carnitine Tablet

### Formulation (percentage by weight):

L-carnitine: 75%
trimetazidine hydrochloride: 0.75%
microcrystalline cellulose: 8%
calcium carbonate: 8%
cross-linked sodium carboxymethyl cellulose: 2.25%
sodium carboxymethyl cellulose: 2%
talcum powder: 3%
magnesium stearate: 1%

### Process:

The same as that in example 9

### Example 12: Compound preparation L-carnitine Tablet

The formulation and process are the same as those in example 9, except that L-carnitine in example 11 is substituted by acetyl-L-carnitine or propionyl-L-carnitine.

### Example 13: Compound preparation L-carnitine Tablet

The formulation and process are the same as those in example 9, except that L-carnitine in example 11 is substituted by a salt formed by L-carnitine and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 14: Compound preparation L-carnitine Tablet

The formulation and process are the same as those in example 9, except that trimetazidine hydrochloride in example 11 is substituted by a salt formed by trimetazidine hydrochloride and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 15: Combined package formulation

Separately prepare or purchase L-carnitine and trimetazidine hydrochloride formulations, as shown in Table 8.

**Table 8 L-carnitine and trimetazidine hydrochloride formulations in different specifications**

| | |
|---|---|
| L-carnitine tablet | trimetazidine hydrochloride tablet |
| L-carnitine tablet 0.25g | trimetazidine hydrochloride tablet 2mg |
| L-carnitine tablet 0.333g | trimetazidine hydrochloride tablet 3mg |
| L-carnitine tablet 0.5g | trimetazidine hydrochloride tablet 5mg |
| L-carnitine tablet 1g | trimetazidine hydrochloride tablet 10mg |
| L-carnitine tablet 2g | trimetazidine hydrochloride tablet 15mg |
| | trimetazidine hydrochloride tablet 20mg |
| | trimetazidine hydrochloride tablet 30mg |

### Example 16: Compound preparation L-carnitine Granule

### Formulation (percentage by weight):

L-carnitine: 8%
trimetazidine hydrochloride: 1%
lactose: 50%
mannitol: 10%
dextrin: 21%
citric acid: 3%
sodium cyclamate: 2%
polyvinylpyrrolidone: 5%

### Process:

1) Let L-carnitine, trimetazidine hydrochloride, lactose, mannitol, polyvinylpyrrolidone, dextrin, citric acid and sodium cyclamate be sifted through 100 mesh sieve, respectively, for further use;
2) Weighing according to the formulation L-carnitine, trimetazidine hydrochloride, lactose, mannitol, polyvinylpyrrolidone, dextrin, citric acid and sodium cyclamate, which are then evenly mixed;
3) Adding an appropriate amount of 70% ethanol solution, preparing soft material and have it sifted through 20 mesh sieve to prepare granules;
4) Placing wet granules in a forced air drier at 50°C and have them dried for four hours;
5) After drying is complete, the granules are taken out and sifted through 20 mesh sieve for granulation.

### Example 17: Compound preparation L-carnitine Granule

### Formulation (percentage by weight):

L-carnitine: 50%
trimetazidine hydrochloride: 0.1%
lactose: 20%
mannitol: 10%
dextrin: 11%
citric acid: 3%
sodium cyclamate: 0.9%
polyvinylpyrrolidone: 5%

### Process:

The same as that in example 16

### Example 18: Compound preparation L-carnitine Granule

### Formulation (percentage by weight):

L-carnitine: 16%
trimetazidine hydrochloride: 0.25%
lactose: 20%
mannitol: 40%
dextrin: 25%
citric acid: 3%
sodium cyclamate: 1%
banana essence: 0.75%
polyvinylpyrrolidone: 4%

### Process:

The same as that in example 16

### Example 19: Compound preparation L-carnitine Granule

### Formulation (percentage by weight):

L-carnitine: 20%
trimetazidine hydrochloride: 0.2%
lactose: 40%
mannitol: 20%
dextrin: 15%
citric acid: 1%
sodium cyclamate: 1%
banana essence: 0.8%
polyvinylpyrrolidone: 2%

### Process:

The same as that in example 16

### Example 20: Compound preparation L-carnitine Granule

The formulation and process are the same as those in example 16, except that L-carnitine in example 19 is substituted by a salt formed by L-carnitine and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 21: Compound preparation L-carnitine Granule

The formulation and process are the same as those in example 16, except that trimetazidine hydrochloride in example 19 is substituted by a salt formed by trimetazidine hydrochloride and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 22: Combined package formulation

Separately prepare or purchase L-carnitine and trimetazidine hydrochloride formulations, as shown in Table 9.

**Table 9 L-carnitine and trimetazidine hydrochloride formulations in different specifications**

| | |
|---|---|
| L-carnitine granule | trimetazidine hydrochloride granule |
| L-carnitine granule 0.25g | trimetazidine hydrochloride granule 2mg |
| L-carnitine granule 0.333g | trimetazidine hydrochloride granule 3mg |
| L-carnitine granule 0.5g | trimetazidine hydrochloride granule 5mg |
| L-carnitine granule 1g | trimetazidine hydrochloride granule 10mg |
| L-carnitine granule 2g | trimetazidine hydrochloride granule 15mg |
| | trimetazidine hydrochloride granule 20mg |
| | trimetazidine hydrochloride granule 30mg |

### Example 23: Compound preparation L-carnitine oral liquid

### Formulation (percentage by weight/volume):

L-carnitine: 5%
trimetazidine hydrochloride: 0.6%
lactose: 10%
mannitol: 10%
citric acid: 5%
sodium cyclamate: 1%
potassium sorbate: 0.02%
distilled water: appropriate

### Process:

Weighing the raw auxiliary material according to the formulation. After being dissolved in an appropriate amount of distilled water, adding more distilled water to dilute it to a predetermined solubility.

### Example 24: Compound preparation L-carnitine oral liquid

### Formulation (percentage by weight/volume):

L-carnitine: 60%
trimetazidine hydrochloride: 0.1%
lactose: 10%
mannitol: 10%
citric acid: 4%
sodium cyclamate: 2%
potassium sorbate: 0.02%
distilled water: appropriate

### Process:

Weighing the raw auxiliary material according to the formulation. After being dissolved in an appropriate amount of distilled water, adding more distilled water to dilute it to a predetermined solubility.

### Example 25: Compound preparation L-carnitine oral liquid

### Formulation (percentage by weight/volume):

L-carnitine: 30%
trimetazidine hydrochloride: 0.3%
lactose: 10%
mannitol: 10%
citric acid: 4%
sodium cyclamate: 2%
potassium sorbate: 0.02%
distilled water: appropriate

### Process:

Weighing the raw auxiliary material according to the formulation. After being dissolved in an appropriate amount of distilled water, adding more distilled water to dilute it to a predetermined solubility.

### Example 26: Compound preparation L-carnitine oral liquid

### Formulation (percentage by weight/volume):

L-carnitine: 10%
trimetazidine hydrochloride: 0.1%
lactose: 5%
mannitol: 15%
citric acid: 2%
sodium cyclamate: 2%
potassium sorbate: 0.02%
distilled water: appropriate

### Process:

Weighing the raw auxiliary material according to the formulation. After being dissolved in an appropriate amount of distilled water, adding more distilled water to dilute it to a predetermined solubility.

### Example 27: Compound preparation L-carnitine oral liquid

The formulation and process are the same as those in example 26, except that L-carnitine in example 26 is substituted by a salt formed by L-carnitine and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 28: Compound preparation L-carnitine oral liquid

The formulation and process are the same as those in example 26, except that trimetazidine hydrochloride in example 26 is substituted by a salt formed by trimetazidine hydrochloride and one of the following: hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid or p-toluene sulfonic acid.

### Example 29: Combined package formulation

Separately prepare or purchase L-carnitine and trimetazidine hydrochloride formulations, as shown in Table 10.

**Table 10 L-carnitine and trimetazidine hydrochloride formulations in different specifications**

| | |
|---|---|
| L-carnitine oral liquid | trimetazidine hydrochloride oral liquid |
| L-carnitine oral liquid 0.25g | trimetazidine hydrochloride oral liquid 2mg |
| L-carnitine oral liquid 0.333g | trimetazidine hydrochloride oral liquid 3mg |
| L-carnitine oral liquid 0.5g | trimetazidine hydrochloride oral liquid 5mg |
| L-carnitine oral liquid 1g | trimetazidine hydrochloride oral liquid 10mg |
| L-carnitine oral liquid 2g | trimetazidine hydrochloride oral liquid 15mg |
| | trimetazidine hydrochloride oral liquid 20mg |
| | trimetazidine hydrochloride oral liquid 30mg |

## Claims

1. An oral pharmaceutical composition, comprising
• active ingredient L-carnitine or a derivative thereof selected from acetyl-L-carnitine and propionyl-L-carnitine, or a pharmaceutically acceptable salt thereof,
• active ingredient trimetazidine or pharmaceutically acceptable salt thereof, and a pharmaceutically acceptable auxiliary material, and
• the weight ratio of L-carnitine or derivative thereof or pharmaceutically acceptable salt thereof and trimetazidine or pharmaceutically acceptable salt thereof is 66-4000:1;
for use in the treatment of clinical manifestations of hypoxia including dizziness, encephalalgia, tinnitus, dim sight, limb asthenia, lower exercise performance, thought slowness, memory deterioration, nausea, vomit, palpitation, brachypnea, tachypnea, fast but weak heart beat, pneumonedema, encephaledema, cerebral apoplexy, shock, respiratory failure, optic nerve injury and cranial nerves injuries.

2. The oral pharmaceutical composition for the use of claim 1, wherein the pharmaceutically acceptable salts of trimetazidine, L-carnitine or derivatives thereof comprise their salts formed with hydrochloric acid, hydrobromic acid, hydroiodic acid, sulfuric acid, nitric acid, phosphoric acid, acetic acid, maleic acid, fumaric acid, citric acid, oxalic acid, succinic acid, tartaric acid, malic acid, mandelic acid, trifluoroacetic acid, pantothenic acid, methane sulfonic acid and p-toluene sulfonic acid.

3. The oral pharmaceutical composition for the use of claim 1 or 2, wherein the oral pharmaceutical composition is a normal tablet, a granule or oral liquid.

4. The oral pharmaceutical composition for the use of claim 3, wherein the oral pharmaceutical composition is a normal tablet; preferably the normal tablet comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

5. The oral pharmaceutical composition for the use of claim 3, wherein the oral pharmaceutical composition is a granule; preferably
the granule comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

6. The oral pharmaceutical composition for the use of claim 3, wherein the oral pharmaceutical composition is oral liquid; preferably the oral liquid comprises active ingredient L-carnitine or derivative thereof or pharmaceutically acceptable salt thereof, active ingredient trimetazidine or pharmaceutically acceptable salt thereof, and a pharmaceutically acceptable auxiliary material, and 100:1 is the weight ratio of L-carnitine or derivative thereof or pharmaceutically acceptable salt thereof and trimetazidine or pharmaceutically acceptable salt thereof.

7. The oral pharmaceutical composition for the use of any one of claims 1-6, wherein the oral pharmaceutical composition is in combined package.

8. The oral pharmaceutical composition for the use of claim 1, wherein the ratio of L-carnitine or derivative thereof or pharmaceutically acceptable salt thereof and trimetazidine or pharmaceutically acceptable salt thereof is 66-100:1.

9. The oral pharmaceutical composition for the use of claim 8, wherein the ratio of L-carnitine or derivative thereof or pharmaceutically acceptable salt thereof and trimetazidine or pharmaceutically acceptable salt thereof is 100:1.

## Patentansprüche

1. Orale pharmazeutische Zusammensetzung, welche umfasst:
• den Wirkstoff L-Carnitin oder ein Derivat davon, ausgewählt aus Acetyl-L-Carnitin und Propionyl-L-Carnitin, oder ein pharmazeutisch anerkanntes Salz davon
• den Wirkstoff Trimetazidin oder ein pharmazeutisch anerkanntes Salz davon und ein pharmazeutisch anerkanntes Hilfsmaterial, und
• das Gewichtsverhältnis von L-Carnitin oder einem Derivat davon oder einem pharmazeutisch anerkannten Salz davon und Trimetazidin oder einem pharmazeutisch anerkannten Salz davon beträgt 66-4000:1;
zur Verwendung bei der Behandlung von klinischen Manifestationen von Hypoxie, einschließlich Schwindel, nervösen Kopfschmerzen (Encephalalgie), Tinnitus, Schwachsichtigkeit, Schwächegefühl (Asthenie) in den Gliedmaßen, abnehmender körperlicher Belastbarkeit, geistiger Trägheit, Gedächtnisproblemen, Übelkeit, Erbrechen, Herzklopfen, Brachypnoe, Tachypnoe, raschem aber schwachem Herzschlag, Lungenödem, Hirnödem, Hirnschlag, Schock, Atemstörungen, Verletzung des Sehnervs und Verletzungen von Hirnnerven.

2. Orale pharmazeutische Zusammensetzung zur Verwendung nach Anspruch 1, wobei die pharmazeutisch anerkannten Salze von Trimetazidin, L-Carnitin oder Derivaten davon ihre mit Salzsäure, Bromwasserstoffsäure, lodwasserstoffsäure, Schwefelsäure, Salpetersäure, Phosphorsäure, Essigsäure, Maleinsäure, Fumarsäure, Citronensäure, Oxalsäure, Bernsteinsäure, Weinsäure, Apfelsäure, Mandelsäure, Trifluoressigsäure, Pantothensäure, Methansulfonsäure und p-Toluolsulfonsäure gebildeten Salze umfassen.

3. Orale pharmazeutische Zusammensetzung zur Verwendung nach Anspruch 1 oder 2, wobei die orale pharmazeutische Zusammensetzung eine normale Tablette, ein Granulat oder eine orale Flüssigkeit ist.

4. Orale pharmazeutische Zusammensetzung zur Verwendung nach Anspruch 3, wobei die orale pharmazeutische Zusammensetzung eine normale Tablette ist, wobei die normale Tablette vorzugsweise den Wirkstoff L-Carnitin oder ein Derivat davon oder ein pharmazeutisch anerkanntes Salz davon, den aktiven Wirkstoff Trimetazidin oder ein pharmazeutisch anerkanntes Salz davon und ein pharmazeutisch anerkanntes Hilfsmaterial umfasst und das Gewichtsverhältnis von L-Carnitin oder dem Derivat davon oder dem pharmazeutisch anerkannten Salz davon und Trimetazidin oder dem pharmazeutisch anerkannten Salz davon 100:1 beträgt.

5. Orale pharmazeutische Zusammensetzung zur Verwendung nach Anspruch 3, wobei die orale pharmazeutische Zusammensetzung ein Granulat ist; wobei das Granulat vorzugweise den Wirkstoff L-Carnitin oder ein Derivat davon oder ein pharmazeutisch anerkanntes Salz davon, den Wirkstoff Trimetazidin oder ein pharmazeutisch anerkanntes Salz davon und ein pharmazeutisch anerkanntes Hilfsmaterial umfasst und das Gewichtsverhältnis von L-Carnitin oder einem Derivat davon oder einem pharmazeutisch anerkannten Salz davon und Trimetazidin oder einem pharmazeutisch anerkannten Salz davon 100:1 beträgt.

6. Orale pharmazeutische Zusammensetzung zur Verwendung nach Anspruch 3, wobei die orale pharmazeutische Zusammensetzung eine orale Flüssigkeit ist, wobei die orale Flüssigkeit vorzugsweise den Wirkstoff L-Carnitin oder ein Derivat davon oder ein pharmazeutisch anerkanntes Salz davon, den Wirkstoff Trimetazidin oder ein pharmazeutisch anerkanntes Salz davon und ein pharmazeutisch anerkanntes Hilfsmaterial umfasst und das Gewichtsverhältnis von L-Carnitin oder einem Derivat davon oder einem pharmazeutisch anerkannten Salz davon und Trimetazidin oder einem pharmazeutisch anerkannten Salz davon 100:1 beträgt.

7. Orale pharmazeutische Zusammensetzung zur Verwendung nach einem der Ansprüche 1-6, wobei die orale pharmazeutische Zusammensetzung in einer Kombinationspackung vorliegt.

8. Orale pharmazeutische Zusammensetzung zur Verwendung nach Anspruch 1, wobei das Verhältnis von L-Carnitin oder einem Derivat davon oder einem pharmazeutisch anerkannten Salz davon und Trimetazidin oder einem pharmazeutisch anerkannten Salz davon 66-100:1 beträgt.

9. Orale pharmazeutische Zusammensetzung zur Verwendung nach Anspruch 8, wobei das Verhältnis von L-Carnitin oder einem Derivat davon oder einem pharmazeutisch anerkannten Salz davon und Trimetazidin oder einem pharmazeutisch anerkannten Salz davon 100:1 beträgt.

## Revendications

1. Composition pharmaceutique orale, comprenant
• un ingrédient actif de type L-carnitine ou un dérivé de celui-ci sélectionné parmi l'acétyl-L-carnitine et la propionyl-L-carnitine, ou un sel pharmaceutiquement acceptable de celui-ci,
• un ingrédient actif de type trimétazidine ou un sel pharmaceutiquement acceptable de celui-ci, et une substance auxiliaire pharmaceutiquement acceptable, et
• le rapport en poids entre la L-carnitine ou un dérivé de celle-ci ou un sel pharmaceutiquement acceptable de celle-ci et la trimétazidine ou un sel pharmaceutiquement acceptable de celle-ci est de 66 à 4000:1 ;
pour son utilisation dans le traitement des manifestations cliniques de l'hypoxie telles que les étourdissements, l'encéphalalgie, les acouphènes, une vue trouble, une asthénie des membres, une performance inférieure à l'exercice, un ralentissement de la pensée, une détérioration de la mémoire, des nausées, des vomissements, des palpitations, une brachypnée, une tachypnée, un rythme cardiaque rapide mais faible, un oedème pulmonaire, un oedème de l'encéphale, une apoplexie cérébrale, un choc, une insuffisance respiratoire, une lésion du nerf optique et les lésions des nerfs crâniens.

2. Composition pharmaceutique orale pour l'utilisation selon la revendication 1, dans laquelle les sels pharmaceutiquement acceptables de trimétazidine, de L-carnitine ou des dérivés de celle-ci comprennent leurs sels formés avec l'acide chlorhydrique, l'acide bromhydrique, l'acide iodhydrique, l'acide sulfurique, l'acide nitrique, l'acide phosphorique, l'acide acétique, l'acide maléique, l'acide fumarique, l'acide citrique, l'acide oxalique, l'acide succinique, l'acide tartrique, l'acide malique, l'acide mandélique, l'acide trifluoroacétique, l'acide pantothénique, l'acide méthane sulfonique et l'acide p-toluène sulfonique.

3. Composition pharmaceutique orale pour l'utilisation selon la revendication 1 ou 2, dans laquelle la composition pharmaceutique orale est un comprimé normal, un granule ou un liquide oral.

4. Composition pharmaceutique orale pour l'utilisation selon la revendication 3, dans laquelle la composition pharmaceutique orale est un comprimé normal ; de préférence le comprimé normal comprend un ingrédient actif de type L-carnitine ou un dérivé de celui-ci ou un sel pharmaceutiquement acceptable de celui-ci, un ingrédient actif de type trimétazidine ou un sel pharmaceutiquement acceptable de celui-ci, et une substance auxiliaire pharmaceutiquement acceptable, et 100:1 est le rapport en poids entre la L-carnitine ou un dérivé de celle-ci ou un sel pharmaceutiquement acceptable de celle-ci et la trimétazidine ou un sel pharmaceutiquement acceptable de celle-ci.

5. Composition pharmaceutique orale pour l'utilisation selon la revendication 3, dans laquelle la composition pharmaceutique orale est un granule ; de préférence le granule comprend un ingrédient actif de type L-carnitine ou un dérivé de celui-ci ou un sel pharmaceutiquement acceptable de celui-ci, un ingrédient actif de type trimétazidine ou un sel pharmaceutiquement acceptable de celui-ci, et une substance auxiliaire pharmaceutiquement acceptable, et 100:1 est le rapport en poids entre la L-carnitine ou un dérivé de celle-ci ou un sel pharmaceutiquement acceptable de celle-ci et la trimétazidine ou un sel pharmaceutiquement acceptable de celle-ci.

6. Composition pharmaceutique orale pour l'utilisation selon la revendication 3, dans laquelle la composition pharmaceutique orale est un liquide oral ; de préférence le liquide oral comprend un ingrédient actif de type L-carnitine ou un dérivé de celui-ci ou un sel pharmaceutiquement acceptable de celui-ci, un ingrédient actif de type trimétazidine ou un sel pharmaceutiquement acceptable de celui-ci, et une substance auxiliaire pharmaceutiquement acceptable, et 100:1 est le rapport en poids entre la L-carnitine ou un dérivé de celle-ci ou un sel pharmaceutiquement acceptable de celle-ci et la trimétazidine ou un sel pharmaceutiquement acceptable de celle-ci.

7. Composition pharmaceutique orale pour l'utilisation selon l'une quelconque des revendications 1 à 6, dans laquelle la composition pharmaceutique orale est dans un emballage combiné.

8. Composition pharmaceutique orale pour l'utilisation selon la revendication 1, dans laquelle le rapport entre la L-carnitine ou un dérivé de celle-ci ou un sel pharmaceutiquement acceptable de celle-ci et la trimétazidine ou un sel pharmaceutiquement acceptable de celle-ci est de 66 à 100:1.

9. Composition pharmaceutique orale pour l'utilisation selon la revendication 8, dans laquelle le rapport entre la L-carnitine ou un dérivé de celle-ci ou un sel pharmaceutiquement acceptable de celle-ci et la trimétazidine ou un sel pharmaceutiquement acceptable de celle-ci est de 100:1.
